# EUROPEAN PATENT APPLICATION

(11) **EP 4 349 380 A1**
(43) Date of publication of application: **10.04.2024**
(21) Application number: 22811000.3
(22) Date of filing: 28.03.2022
(51) Int. Cl.: A61M 1/14, A61L 9/00

(54) **BLOOD PURIFICATION DEVICE AND BLOOD PURIFICATION MANAGEMENT SYSTEM**

(30) Priority: 28.05.2021 JP 2021090054
(71) Applicant: Nikkiso Co., Ltd., Tokyo 150-6022 (JP)
(72) Inventor: OBATA, Yuki, Makinohara-shi, Shizuoka 421-0496 (JP)
(74) Representative: von Hirschhausen, Helge
(86) International application number: PCT/JP2022/015005
(87) International publication number: WO 2022/249724

(57) **Abstract**

An object is to provide a blood purification apparatus and a blood purification management system that can purify an ambient environment reliably and in a simple and easy manner in a case of providing blood purification treatment.

A blood purification apparatus 100 including a blood purification unit by including a dialyzer, a blood pump 123, and the like includes an air purification unit that discharges external air sucked by an intake air fan 16 to inside of an upper space 101 and a lower space 103 in which printed substrates 153 and 155 are disposed after purification by a purification unit 15, and a control unit 150 purifies ambient air in conjunction with blood purification processing of a patient.

## Description

### Technical Field

The present invention relates to a blood purification apparatus and a blood purification management system that can purify the air in an ambient environment.

### Background Art

A treatment facility needs to be soundness as an environment to use a medical device, and particularly in a case of providing treatment for a long time, someone's health must never be deteriorated. For example, since purification treatment of blood, which is so-called dialysis treatment, takes time, an ambient environment needs to be comfortable, and it is necessary to collect dust floating in the air and also to sterilize or purify viruses, bacteria, and the like floating with the dust.

Therefore, for example, in a treatment facility in which a dialysis apparatus as described in PTLs 1 and 2 is disposed, it is necessary to dispose an air purification apparatus as described in PTLs 3 and 4.

### Citation List

### Patent Literature

PTL 1: Japanese Patent Laid-Open No. 2021-010522
PTL 2: Japanese Patent Laid-Open No. 2021-049061
PTL 3: Japanese Patent Laid-Open No. 2005-342509
PTL 4: Japanese Patent Laid-Open No. 2010-075418

### Summary of Invention

### Technical Problem

It is cumbersome to dispose such blood purification apparatus and air purification apparatus in a treatment facility and operate them separately, and it is troublesome to drive both of them in a reliable and comfortable manner. For this reason, there is a possibility that the purification is not performed in the dialysis treatment.

However, there is a need to obviate the occurrence of a viral disease that occurs frequently in recent years, and it is necessary to secure a treatment environment that can reliably avoid particularly a patient undergoing the dialysis treatment being affected.

To this end, an object of the present invention is to provide a blood purification apparatus and a blood purification management system that can purify an ambient environment reliably and in a simple and easy manner in a case of providing the blood purification treatment.

### Solution to Problem

An aspect of the invention of a blood purification apparatus that solves the above-mentioned problem is a blood purification apparatus including a blood purification unit that executes blood purification treatment, including: an air purification unit that sucks external air and discharges the sucked external air after purification; and a control unit that controls the blood purification unit and the air purification unit, in which the control unit drives the air purification unit in conjunction with running of the blood purification unit and allows the air purification unit to purify air around a patient subjected to the purification of blood by the blood purification unit.

An aspect of the invention of a blood purification management system that solves the above-mentioned problem is a system to which multiple blood purification apparatuses described above are connected and that manages a running schedule, in which a control signal to drive the air purification unit is transmitted to one or more separate blood purification apparatuses disposed around the blood purification apparatus in which the blood purification unit runs.

### Advantageous Effects of Invention

According to an aspect of the present invention as described above, it is possible to purify an ambient environment reliably and in a simple and easy manner in a case of providing blood purification treatment, and it is possible to secure a clean treatment facility and to provide a high-quality treatment environment.

### Brief Description of Drawings

[Fig. 1] Fig. 1 is a diagram illustrating a first embodiment of a blood purification apparatus according to the present invention and is a plan view illustrating an example of a disposing environment thereof.
[Fig. 2] Fig. 2 is a perspective view illustrating an exterior appearance of the blood purification apparatus.
[Fig. 3] Fig. 3 is a block diagram illustrating a processing system of driving control of the blood purification apparatus.
[Fig. 4] Fig. 4 is a transparent side view illustrating an air purification function provided in the blood purification apparatus.
[Fig. 5] Fig. 5 is a timing chart describing air purification processing in a case where blood purification processing thereof is performed in the morning and in the afternoon.
[Fig. 6] Fig. 6 is a timing chart describing the air purification processing in a case where the blood purification processing thereof is performed in the morning.
[Fig. 7] Fig. 7 is a timing chart describing the air purification processing in a case where the blood purification processing thereof is performed in the afternoon.
[Fig. 8] Fig. 8 is a transparent side view illustrating an air-cooling function provided in the blood purification apparatus.
[Fig. 9] Fig. 9 is a perspective view illustrating an air purification apparatus included in a second embodiment of the blood purification apparatus according to the present invention.
[Fig. 10] Fig. 10 is a diagram illustrating a third embodiment of the blood purification apparatus according to the present invention, while Fig. 10A is a perspective view illustrating an air purification apparatus included therein, and Fig. 10B is a perspective view illustrating an attachment metal fitting thereof.
[Fig. 11] Fig. 11 is an elevation view including a bed of a patient that illustrates an air purification unit of a fourth embodiment of the blood purification apparatus according to the present invention.
[Fig. 12] Fig. 12 is an elevation view including the bed of the patient that illustrates an air purification unit of a fifth embodiment of the blood purification apparatus according to the present invention.
[Fig. 13] Fig. 13 is a plan view including the vicinity of the bed of the patient that illustrates an air purification unit of a sixth embodiment of the blood purification apparatus according to the present invention.
[Fig. 14] Fig. 14 is an enlarged view illustrating wind direction adjustment provided in an air purification unit of a seventh embodiment of the blood purification apparatus according to the present invention.
[Fig. 15] Fig. 15 is an enlarged view illustrating wind direction adjustment provided in an air purification unit of an eighth embodiment of the blood purification apparatus according to the present invention.
[Fig. 16] Fig. 16 is a block diagram illustrating a processing system of driving control of a ninth embodiment of the blood purification apparatus according to the present invention.
[Fig. 17] Fig. 17 is a diagram illustrating an embodiment of a blood purification management system according to the present invention and is a plan view illustrating an example of a disposing environment thereof.
[Fig. 18] Fig. 18 is a timing chart describing timings of blood purification processing and air purification processing thereof.

### Description of Embodiments

Embodiments of the present invention are described below in detail with reference to the drawings.

### <First Embodiment>

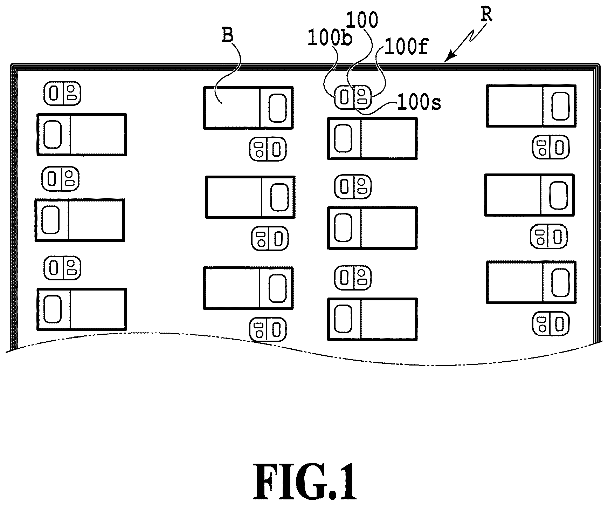

Figs. 1 to 7 are diagrams illustrating a first embodiment of a blood purification apparatus according to the present invention.

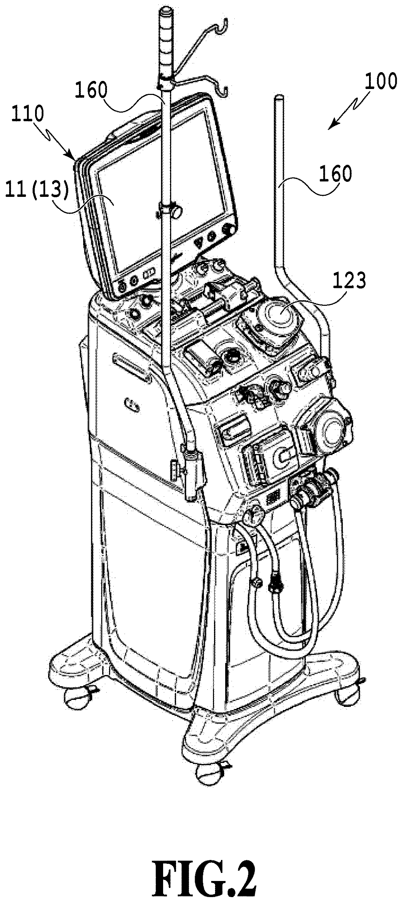

In Figs. 1 and 2, a blood purification apparatus 100 is disposed and prepared beside a head Ph side of a patient P so as to be able to provide dialysis treatment (blood purification processing) at each bed B of the patient P and is arranged such that an apparatus front surface 100f on a front surface side of an operation display unit 110 faces in a direction of a foot Pf side of the patient P. Here, in the present embodiment, a dialysis facility (treatment room) R in which the multiple beds B for the patients P are arranged is described as an example; however, it is not limited thereto, and it is needless to say that it is applicable also in a case in which one bed is disposed in each room, and it is applicable also in a case of a chair with a reclining function and the like, for example.

This blood purification apparatus 100 includes a blood purification unit that performs dialysis processing by drive-controlling a blood pump 123 and a dual pump, a water removal pump, and the like (not illustrated) according to setting prescribed in advance or obtained various types of sensor information, the blood pump 123 pumping blood of the patient P so as to draw and circulate the blood of the patient P in a blood circuit passing through a not-illustrated dialyzer and thereafter return the blood, and the dual pump, the water removal pump, and the like pumping a dialysate so as to take the dialysate into a dialysis circuit passing through the dialyzer and drain after the dialysis processing.

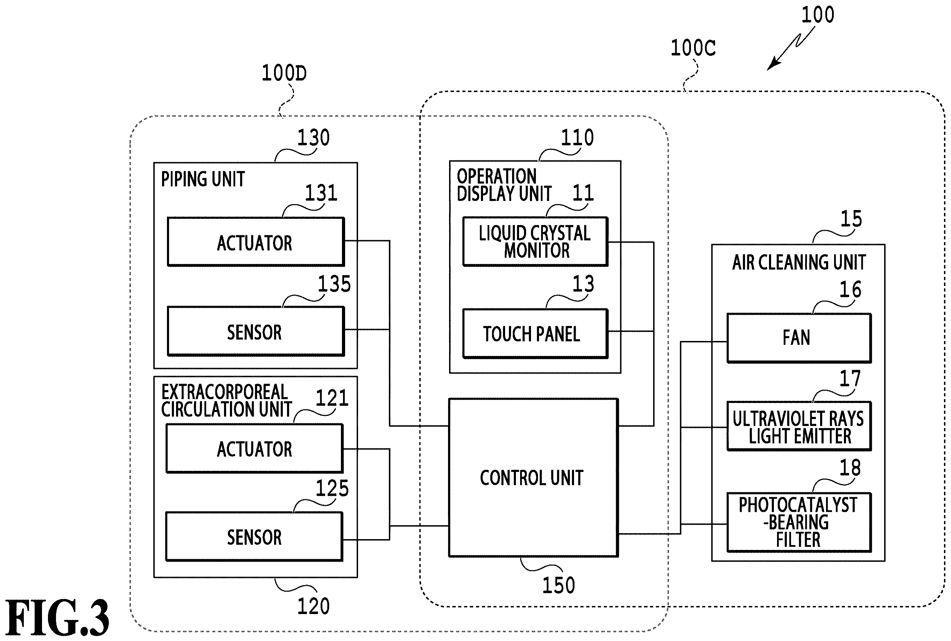

Additionally, as illustrated in a simple block diagram in Fig. 3, in the blood purification apparatus 100, the operation display unit 110, an extracorporeal circulation unit 120, and a piping unit 130 are connected to a control unit 150 so as to be controlled overall. The operation display unit 110 is constructed by including a liquid crystal monitor 11 that display-outputs various types of information such as a treatment state and a schedule and a touch panel 13 that performs an input operation of various types of information such as treatment conditions and the schedule. The extracorporeal circulation unit 120 is constructed by including an actuator group 121 that includes the blood pump 123, a valve (not illustrated), and the like that flow the blood of the patient P into the blood circuit and a sensor group 125 that detects various types of information such as a pressure of the blood in the blood circuit. The piping unit 130 is constructed by including an actuator group 131 that includes the dual pump, a valve, and the like that flow a fluid such as the dialysate into the dialysis circuit and a sensor group 135 that detects various types of information such as a temperature, a pressure, and the like of the dialysate in the dialysis circuit. The control unit 150 provides the patient P with the dialysis treatment with a CPU executing control processing according to various types of sensor information and various types of setting information based on a control program stored in a memory and controlling and adjusting the driving of the operation display unit 110, the extracorporeal circulation unit 120, and the piping unit 130.

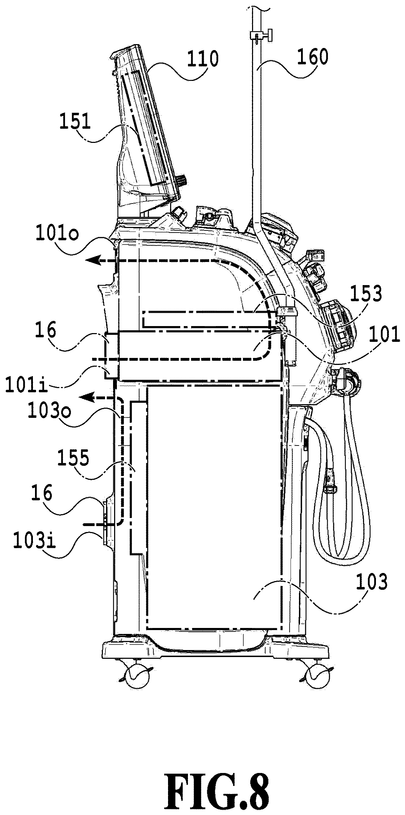

As illustrated in a conventional structure in Fig. 8, in the control unit 150, the CPU that executes control processing, various memories, and the like are mounted on a printed substrate 151 and stored in the operation display unit 110 of an apparatus main body of the blood purification apparatus 100, and a control circuit and the like that execute input and output processing of the liquid crystal monitor 11 and the touch panel 13 of the operation display unit 110 are also mounted on this printed substrate 151. Additionally, in the control unit 150, a printed substrate 153 and a printed substrate 155 of a power unit on which a power source unit and the like adjusting an electric power amount and the like to be supplied to a driver of various sensors and a driving mechanism such as various motors or valves, for example, are connected to the printed substrate 151 and controlled overall by the CPU and the like are stored in an upper space 101 and on a back surface side of a lower space 103 of the apparatus main body of the blood purification apparatus 100.

In this control unit 150, since heating occurs due to electric power supply to the various drivers and driving mechanisms with the information processing by the CPU and the like, the printed substrates (heating portions) 153 and 155 are set in the upper space 101 and on the back surface side of the lower space 103 securing a spatial capacity in order to secure running within a desired temperature range that never exceeds an upper limit temperature to implement control processing with no malfunction. Additionally, this control unit 150 is provided with a cooling function to flow external air into the upper space 101 and the lower space 103 in order to suppress a temperature rise in the upper space 101 and the lower space 103 in a ventilation route in which the printed substrates 153 and 155 are set. As this cooling function, intake air ports 101i, 103i positioned at lower portions of the upper space 101 and the lower space 103 and opening in an apparatus back surface 100b and exhaust air ports 101o, 103o positioned at upper portions thereof and opening in the apparatus back surface 100b are arranged, and out of those, intake air fans 16 are disposed in the intake air ports 101i, 103i.

Thus, as indicated by an arrow in Fig. 8, the blood purification apparatus 100 can suppress a temperature rise around the printed substrates 153 and 155 by forcibly flowing internal air at a temperature raised inside the upper space 101 and the lower space 103 out of the upper exhaust air ports 101o, 103o while flowing the relatively low external air from the lower intake air ports 101i, 103i to take as cooling air.

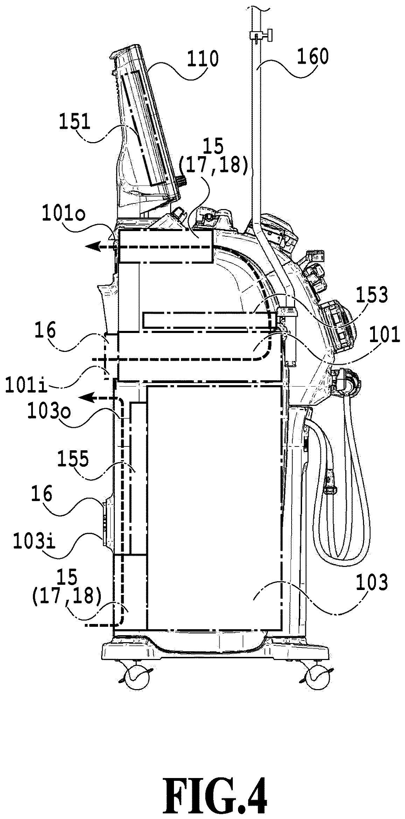

Additionally, as illustrated in Fig. 4, in the blood purification apparatus 100, in one of the intake air ports 101i, 103i and one of the exhaust air ports 101o, 103o in the upper space 101 and the lower space 103, a purification unit 15 that purifies the flowing air passing therethrough is disposed in connection with the printed substrates 153 and 155 so as to be drivable together with the intake air fans 16. The purification unit 15 is configured to perform inactivation processing while collecting dust included in the flowing air and to flow the purified exhaust air to the outside. For example, as illustrated in Fig. 3, this purification unit 15 includes a filter 18 bearing a photocatalyst to collect the dust and a light emitter 17 that irradiates the filter 18 with ultraviolet rays to function the photocatalyst, and has a function to sterilize and inactivate viruses, bacteria, and the like included in the flowing air in the internal spaces of the upper space 101 and the lower space 103. That is, the blood purification apparatus 100 includes an air purification unit 100C together with a dialysis function (blood purification unit) 100D. Here, in the present embodiment, the purification unit 15 including the photocatalyst-bearing filter 18 and the ultraviolet rays light emitter 17 is described as an example; however, it is not limited thereto, and it is needless to say that a configuration provided with the function of sterilization and inactivation by another method may be employed.

Additionally, the blood purification apparatus 100 is provided with a function of setting the control unit 150 by using the liquid crystal monitor 11 and the touch panel 13 of the operation display unit 110 to start driving and run in conjunction with the power-on of the intake air fans 16 of the intake air ports 101i, 103i and the purification unit 15.

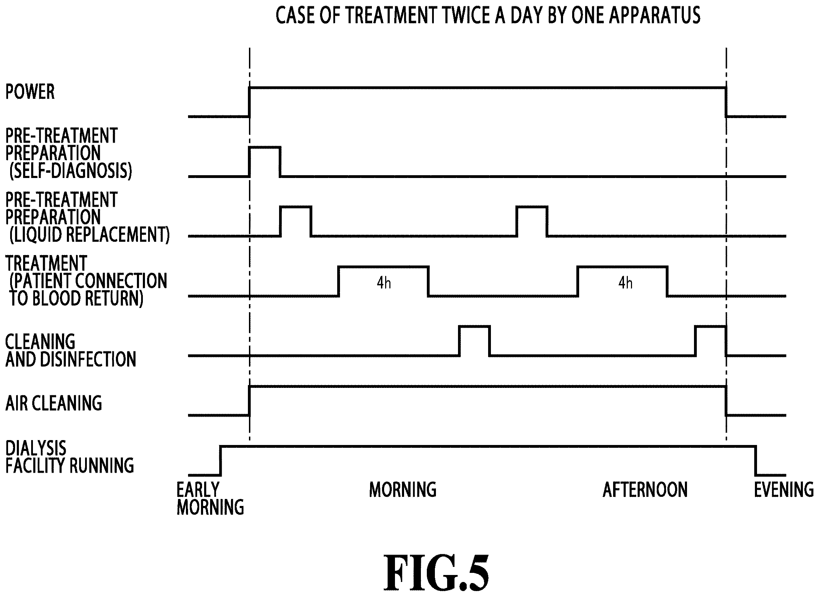

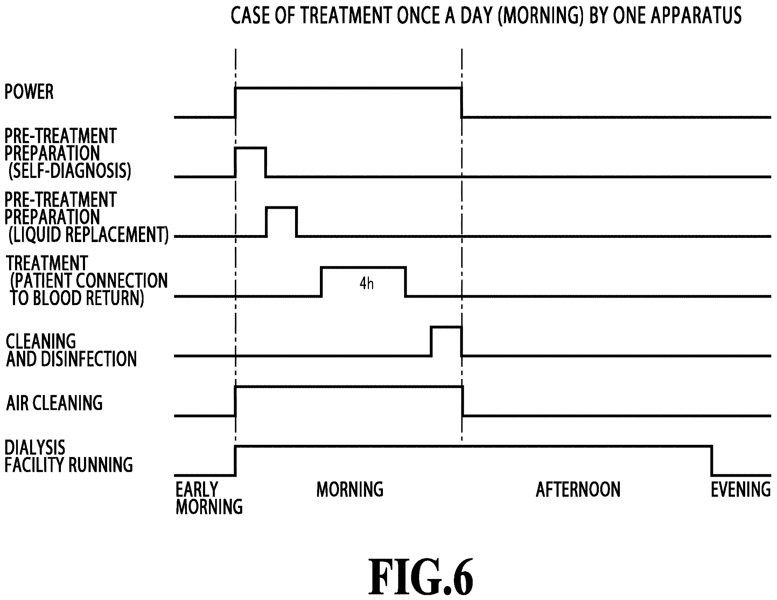

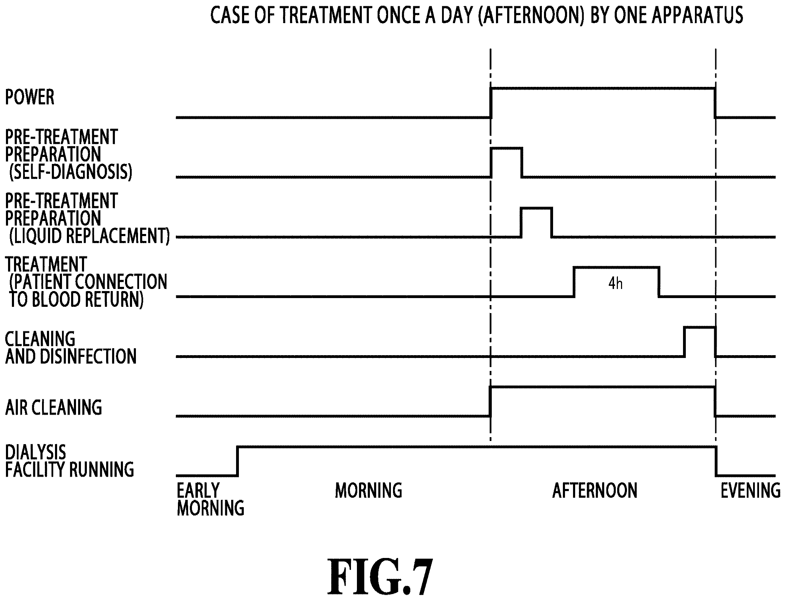

In the control unit 150, various types of processing required at the time in accordance with a schedule of the dialysis treatment is executed while the blood purification apparatus 100 is powered on (ON) in the early morning and shut off (OFF) in the evening in a period of time of the running from opening to closing of the dialysis facility R. For example, as illustrated in Fig. 5, in a case where the dialysis treatment is provided to one patient in the morning and one in the afternoon, the various types of required processing in accordance with the schedule of the dialysis treatment is executed between the power-on (ON) of the blood purification apparatus 100 in the early morning and the shut-off (OFF) in the evening. Additionally, as illustrated in Fig. 6, in a case of providing the dialysis treatment to one patient in the morning, after the power-on (ON) of the blood purification apparatus 100 in the early morning, the various types of processing required for the dialysis treatment is executed. Moreover, as illustrated in Fig. 7, in a case of providing the dialysis treatment to one patient in the afternoon, after the power-on (ON) of the blood purification apparatus 100 in the afternoon, the various types of processing required before the afternoon dialysis treatment is executed. Note that, as the schedule of the dialysis treatment, the control unit 150 may be configured to be able to make a running appointment within a desired period of time that is not only one day but also few days, one week, one month, and the like.

Specifically, the control unit 150 is configured to start the purification processing of the air around the bed B in use and continue it until the power is shut off by driving the purification unit 15 together with the intake air fans 16 of the intake air ports 101i, 103i in parallel with starting self-diagnosis to confirm the soundness of each apparatus unit of the blood purification apparatus 100 concurrently with the power-on and completing it before the dialysis treatment. Then, once completing the self-diagnosis as pre-treatment preparation of the blood purification apparatus 100, the control unit 150 performs processing of replacing the inside of the dialysis circuit with the dialysate and thereafter provides the dialysis treatment in which the blood is returned to the patient P with about four hours of the dialysis processing through the blood circuit and the dialysis circuit connected by puncture of a blood vessel of the patient P. The control unit 150 is configured to perform cleaning and disinfection processing of the dialysis circuit thereafter, and after completing the cleaning and disinfection processing along with this dialysis treatment for one day, the driving of the purification unit 15 is automatically stopped with the intake air fans 16 of the intake air ports 101i, 103i, the power to the blood purification apparatus 100 is shut off, and the series of the dialysis treatment processing ends. Note that, the blood purification apparatus 100 may be configured to not only drive the dialysis processing and the air purification function in conjunction with each other by using the operation display unit 110 but also input setting for driving uniquely in a desired timing, wind blowing amount, and light intensity, respectively, and it is favorable to be able to grasp the state by display-outputting a driving state of the dialysis processing, the air purification processing, and the like.

Thus, the blood purification apparatus 100 can suck the external air around the head Ph of the patient P on each bed B to replace with the purified air with no time and effort of disposing and operating a special individual air purification apparatus and also in conjunction with the dialysis processing without wasteful electric power consumption, and can provide the dialysis treatment in an ambient environment with the purified air subjected to the sterilization and inactivation processing of viruses and bacteria.

### <Effect of First Embodiment>

Thus, in the blood purification apparatus 100 of the present embodiment, it is possible to be provided with a purification function by diverting a cooling function of the control unit 150, and it is possible to efficiently prevent the infection of the patient P undergoing the dialysis treatment with viruses and bacteria in a simple and easy manner and to avoid the spread of an infectious disease from the patient P. Accordingly, it is possible to purify the dialysis treatment environment around the patient P at high quality by employing the blood purification apparatus 100.

### <Second Embodiment>

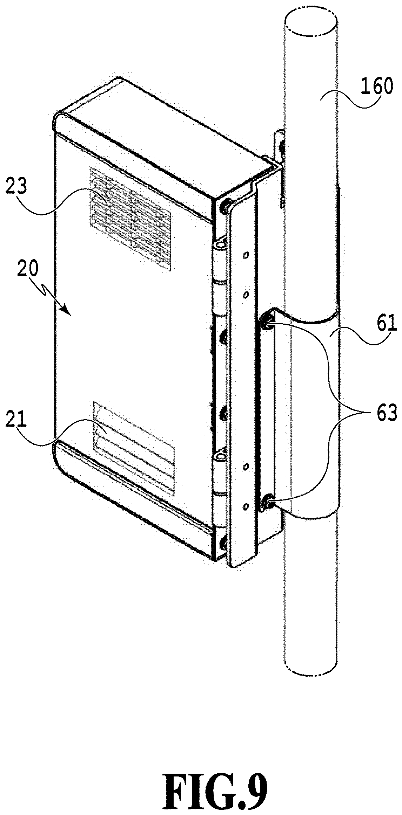

Fig. 9 is a diagram illustrating a second embodiment of the blood purification apparatus according to the present invention. Here, since the present embodiment has substantially the same configuration as that of the above-described embodiment, characteristic portions are described diverting the drawings and using the common reference signs (the same applies to other embodiments described below).

In Fig. 9, the blood purification apparatus 100 includes a support pole 160 (see Fig. 2) from which saline is hung and to which an auxiliary device such as an infusion pump is attached, and in the present embodiment, instead of the purification unit 15 in the above-described embodiment, there is included an air purification apparatus 20 in a box shape in which a purification unit that can be attached and supported on one of the right and left support poles 160 with an attachment metal fitting 61 and an attachment screw 63 is mounted. Here, the attachment metal fitting 61 is fixed tightly to the support pole 160 by being set to a state of winding to tighten the support pole 160 and being screwed onto a housing of the air purification apparatus 20 with the attachment screw 63. Note that, this mode of attaching the air purification apparatus 20 is not limited thereto, and it is needless to say that another attachment means may be used.

As with the above-described embodiment, the air purification apparatus 20 includes an intake air port 21 and an exhaust air port 23 and is configured to be able to add the function of sterilizing and inactivating viruses, bacteria, and the like included in the flowing external air to the blood purification apparatus 100 by collecting the viruses, bacteria, and the like by the photocatalyst-bearing filter 18 forming the purification unit while sucking and discharging the external air flowing through an internal space in the housing with a not-illustrated fan, and functioning the photocatalyst by emitting the ultraviolet rays from the light emitter 17.

This air purification apparatus 20 is connected with wire (wireless connection may be applicable) to the control unit 150 of the blood purification apparatus 100, and as with the above-described embodiment, it is possible to attach and run the air purification apparatus 20 on either right or left beside the bed B of the patient P undergoing the dialysis treatment with no limitation and to set so as to start driving in conjunction with the running of the blood purification apparatus 100, and it is needless to say that, not only the driving in conjunction with the blood purification apparatus 100, the air purification apparatus 20 can be driven uniquely in a desired timing.

### <Effect of Second Embodiment>

Thus, in the blood purification apparatus 100 of the present embodiment, it is possible to obtain the operation and effect similar to that of the above-described embodiment, and it is possible to implement purification of the dialysis treatment environment around the patient P more simply, easily, and inexpensively. Additionally, since it is possible to easily change an attachment position of the air purification apparatus 20, it is possible to flexibly set an air purification region in accordance with right and left arms to be punctured and a body position of the patient and to effectively purify the environment around the patient P.

### <Third Embodiment>

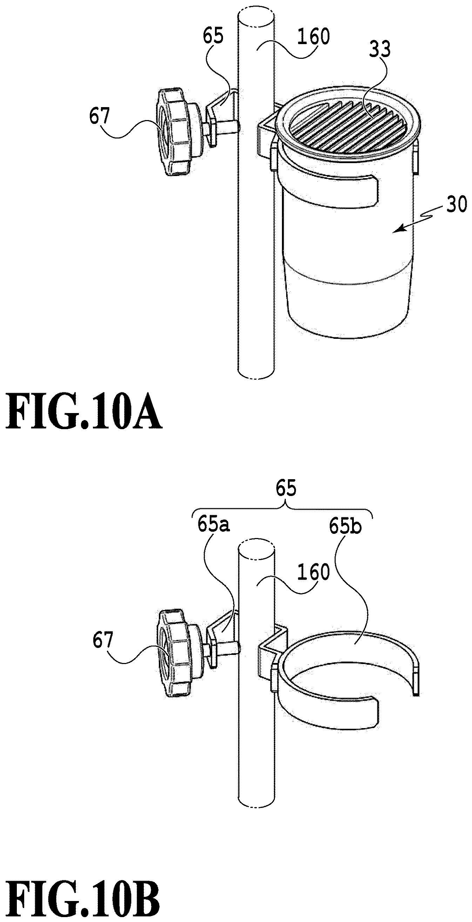

Fig. 10 is a diagram illustrating a third embodiment of the blood purification apparatus according to the present invention.

In Fig. 10, instead of the air purification apparatus 20 in the above-described second embodiment, the blood purification apparatus 100 includes an air purification apparatus 30 in a cylindrical shape in which a purification unit that can be attached and supported on one of the right and left support poles 160 with an attachment metal fitting 65 and an attachment screw 67 is mounted. Here, as illustrated in Fig. 10B, the attachment metal fitting 65 includes a screwing portion 65a and a support ring portion 65b so as to be able to be fixed tightly to the support pole 160 with the screwing portion 65a being set to a state of storing the support pole 160 and a tip of a the screwed and hand-turned screw 67 being brought into contact with an outer surface of the support pole 160, and as illustrated in 10A, the air purification apparatus 30 in the cylindrical shape is inserted into this support ring portion 65b to be supported. Note that, this mode of attaching the air purification apparatus 30 is not limited thereto, and it is needless to say that another attachment means may be used.

As with the air purification apparatus 20 of the above-described embodiment, the air purification apparatus 30 is configured to suck the external air from a not-illustrated intake air port opening in a lower portion and to discharge the air from an exhaust air port 33 in an upper portion after sterilizing and inactivating viruses, bacteria, and the like included in the flowing external air.

As with the air purification apparatus 20 of the above-described embodiment, this air purification apparatus 30 is also connected with wire (wireless connection may be applicable) to the control unit 150 of the blood purification apparatus 100, it is possible to attach and run the air purification apparatus 30 on either right or left beside the bed B of the patient P undergoing the dialysis treatment with no limitation and to set so as to start driving in conjunction with the running of the blood purification apparatus 100, and it is needless to say that, not only this driving in conjunction with the blood purification apparatus 100, the air purification apparatus 30 can be driven uniquely in a desired timing.

### <Effect of Third Embodiment>

Thus, in the blood purification apparatus 100 of the present embodiment, it is possible to obtain the operation and effect similar to that of the above-described second embodiment by using the air purification apparatus 30 that is a separated body by, for example, only updating a control program of the control unit 150 without changing a design of a main body structure of the blood purification apparatus 100, and it is possible to implement purification of the dialysis treatment environment around the close vicinity of the patient P more simply, easily, and inexpensively.

### <Fourth Embodiment>

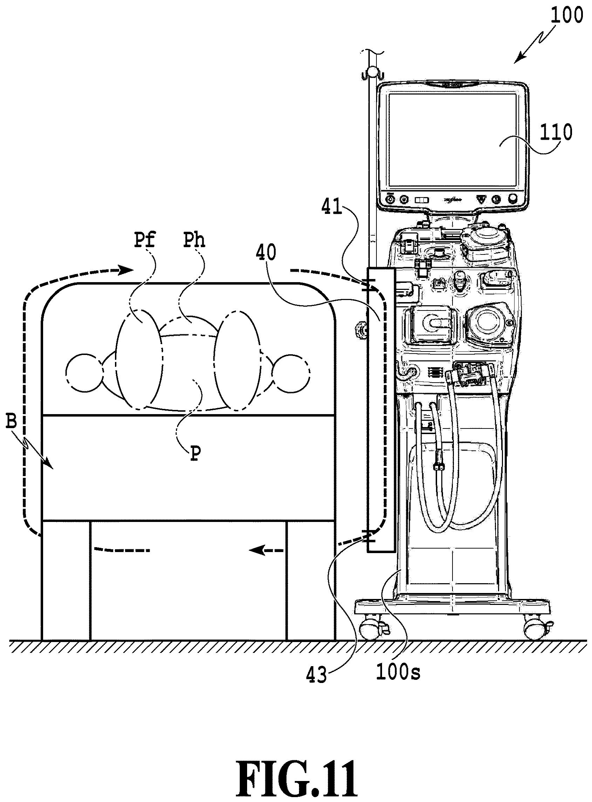

Fig. 11 is a diagram illustrating a fourth embodiment of the blood purification apparatus according to the present invention.

In Fig. 11, in the blood purification apparatus 100, a flow route unit 40 in which a ventilation flow route communicating with the intake air ports 101i, 103i and the exhaust air ports 101o, 103o in the upper space 101 and the lower space 103 (may communicate with only one of them) and extending to the outside to is laid out is attached to an apparatus side surface 100s.

The flow route unit 40 includes an aggregated intake air port 41 communicating with the intake air ports 101i, 103i and an aggregated exhaust air port 43 communicating with the exhaust air ports 101o, 103o. As indicated by an arrow in Fig. 11, for example, the aggregated intake air port 41 is positioned at a height of about 90 cm from a floor surface so as to suck the air around the head Ph of the patient P undergoing the dialysis treatment and lying on the bed B. As indicated by an arrow in Fig. 11, for example, the aggregated exhaust air port 43 is laid out to be positioned near the floor surface such that the exhaust air reaches the opposite side from under the same bed B while not interfering the flow along the intake air. Here, in the present embodiment, a case of disposing on the apparatus left side surface 100s is described as an example; however, it is needless to say that the ventilation flow route may be laid out to be disposable on an apparatus right side surface on the opposite side.

Thus, the blood purification apparatus 100 can aggressively suck the expired air exhaled from the patient P lying on the bed B from the intake air port 41 positioned at a height of the head Ph of the patient P, can discharge the air after the sterilization and inactivation to purify viruses, bacteria, and the like that may be included in a cough, a sneeze, a conversation, an expired air, and the like thereof, and can form a flow of the circulating air by discharging under the bed B the purified exhaust air from the exhaust air port 43 in the lower portion.

### <Effect of Fourth Embodiment>

Thus, in the blood purification apparatus 100 of the present embodiment, in addition to the operation and effect similar to that of the above-described embodiment, it is possible to forcibly suck the expired air of the patient P and the like and discharge the air after the purification processing, and it is possible to implement aggressive purification of the dialysis treatment environment around the patient P.

### <Fifth Embodiment>

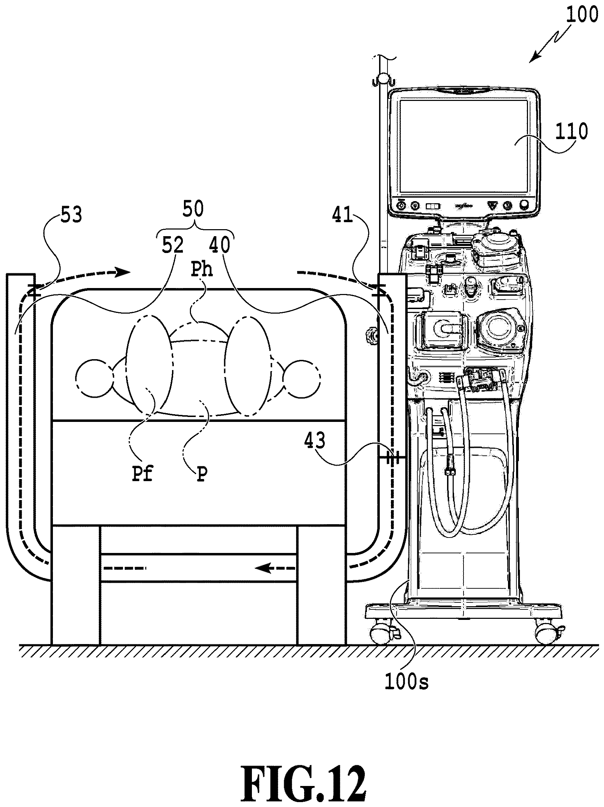

Fig. 12 is a diagram illustrating a fifth embodiment of the blood purification apparatus according to the present invention.

In Fig. 12, in the blood purification apparatus 100, in addition to the flow route unit 40 of the above-described embodiment, a flow route unit 50 that similarly includes the aggregated intake air port 41 and an extending exhaust air port 53 is disposed.

In the flow route unit 50, an exhaust air duct 52 that passes under the bed B to stand up from the opposite side to extend the ventilation flow route to the extending exhaust air port 53 at a height facing the aggregated intake air port 41 and that discharges the air to the outside is continuously attached to the aggregated exhaust air port 43. Here, in the present embodiment, a case of disposing on the apparatus left side surface 100s is described as an example; however, it is needless to say that the ventilation flow route may be laid out to be disposable on the apparatus right side surface on the opposite side, and also, it may be disposable on an intake air port side, and additionally, the duct may be deformable so as to be able to arbitrarily adjust an extending position.

Thus, as indicated by an arrow in Fig. 12, the flow route unit 50 of the blood purification apparatus 100 can position the extending exhaust air port 53 of the exhaust air duct 52 near the head Ph of the patient P undergoing the dialysis treatment and lying on the bed B to forcibly circulate the expired air of the patient P and the like and can aggressively provide the purified air subjected to the sterilization and inactivation.

### <Effect of Fifth Embodiment>

Thus, in the blood purification apparatus 100 of the present embodiment, in addition to the operation and effect similar to that of the above-described embodiment, it is possible to forcibly suck the expired air of the patient P and the like and to discharge the purified air subjected to the purification processing around the patient P, and it is possible to implement further aggressive purification of the dialysis treatment environment.

### <Sixth Embodiment>

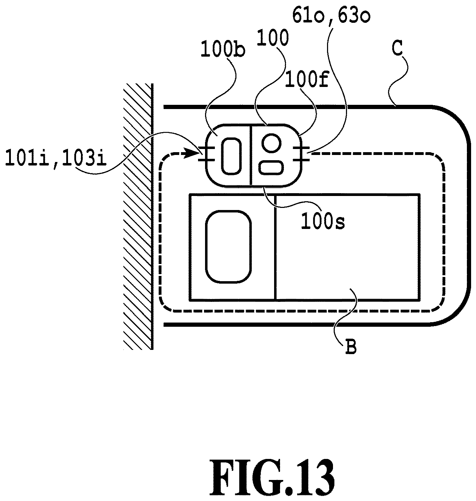

Fig. 13 is a diagram illustrating a sixth embodiment of the blood purification apparatus according to the present invention.

In Fig. 13, in the blood purification apparatus 100, instead of the exhaust air ports 101o, 103o in the upper space 101 and the lower space 103 in the above-described embodiment, exhaust air ports 61o, 63o in the upper space 101 and the lower space 103 are arranged on the apparatus front surface 100f. Here, in the present embodiment, a case of arranging the intake air port and the exhaust air port in the front and rear of the apparatus is described as an example; however, it is not limited thereto, and it is needless to say that the intake air port and the exhaust air port may be arranged on the right and left of the apparatus, for example.

Thus, in a case where a shielding curtain C is disposed around the bed B of the patient P undergoing the dialysis treatment in the dialysis facility R, the blood purification apparatus 100 can discharge the external air, which is sucked from the intake air ports 101i, 103i on the apparatus back surface 100b side, from the exhaust air ports 61o, 63o on the apparatus front surface 100f side after the purification processing. Therefore, the exhaust air can go around in a horizontal direction along an inner side of the curtain C around the bed B to reach the apparatus back surface 100b side, and it is possible to circulate the air in a space partitioned by the curtain C and to purify the treatment environment repeatedly.

### <Effect of Sixth Embodiment>

Thus, in the blood purification apparatus 100 of the present embodiment, in addition to the operation and effect similar to that of the above-described embodiment, it is possible to forcibly perform the purification processing of the air in the treatment environment by using the curtain C partitioning the treatment environment of the patient P, and it is possible to implement aggressive purification of the dialysis treatment environment around the patient P.

### <Seventh Embodiment>

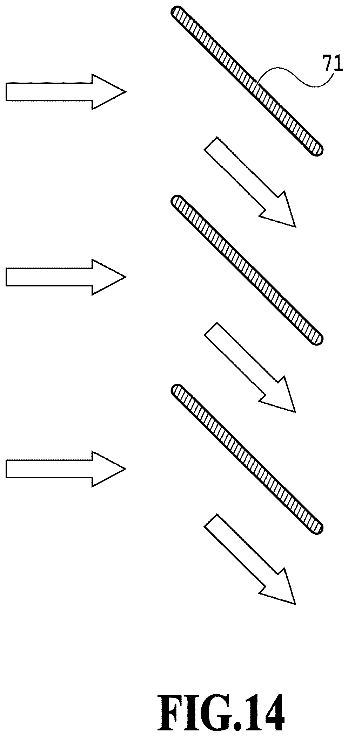

Fig. 14 is a diagram illustrating a seventh embodiment of the blood purification apparatus according to the present invention.

In Fig. 14, in the blood purification apparatus 100, for example, a wind direction plate 71 that changes a wind direction is disposed in one of or both the intake air port and exhaust air port in the above-described embodiment so as to be able to adjust the flow of the external air sucked and discharged into and from the inside of the apparatus by the wind direction plate 71 to set the sucked and discharged air to a targeted space.

Thus, for example, with the wind direction plate 71, the blood purification apparatus 100 can aggressively suck and discharge the external air in a space at a height of the bed B on which the patient P is lying and at a desired height in accordance with a treatment chair and the like on which the patient P is sitting and can secure the purified treatment environment.

### <Effect of Seventh Embodiment>

Thus, in the blood purification apparatus 100 of the present embodiment, in addition to the operation and effect similar to that of the above-described embodiment, it is possible to perform the purification processing while targeting a desired position in the treatment environment by disposing the wind direction plate 71 to the intake air port and the exhaust air port, and it is possible to implement aggressive purification of the dialysis treatment environment around the patient P.

### <Eighth Embodiment>

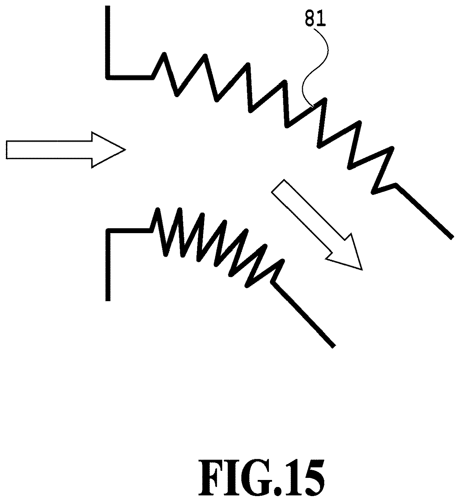

Fig. 15 is a diagram illustrating an eighth embodiment of the blood purification apparatus according to the present invention.

In Fig. 15, in the blood purification apparatus 100, for example, instead of the wind direction plate 71 in the above-described embodiment, a bellows 81 that changes a wind direction is disposed in one of or both the intake air port and exhaust air port so as to be able to adjust the flow of the external air sucked and discharged into and from the inside of the apparatus by the bellows 81 to set the sucked and discharged air to a targeted space.

Thus, for example, with the bellows 81, the blood purification apparatus 100 can aggressively suck and discharge the external air in the space at the height of the bed B on which the patient P is lying and at a desired height in accordance with the treatment chair and the like on which the patient P is sitting and can secure the purified treatment environment.

### <Effect of Eighth Embodiment>

Thus, in the blood purification apparatus 100 of the present embodiment, in addition to the operation and effect similar to that of the above-described embodiment, it is possible to perform the purification processing while targeting a desired position in the treatment environment by disposing the bellows 81 to the intake air port and the exhaust air port, and it is possible to implement aggressive purification of the dialysis treatment environment around the patient P.

### <Ninth Embodiment>

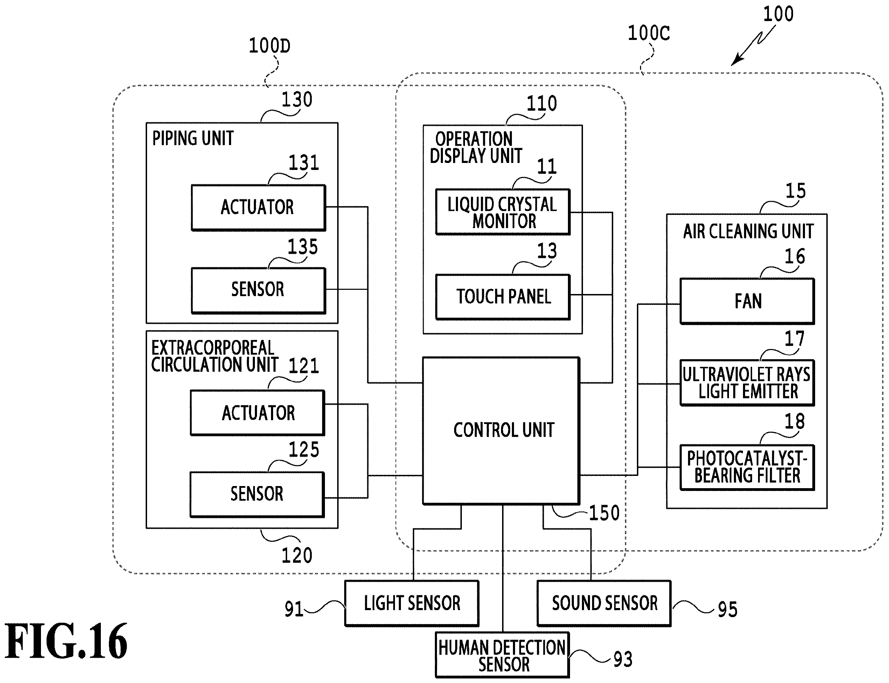

Fig. 16 is a diagram illustrating a ninth embodiment of the blood purification apparatus according to the present invention.

In Fig. 16, in the blood purification apparatus 100, a light sensor 91, a human detection sensor 93, and a sound sensor 95 are connected to the control unit 150 so as to be able to detect various types of sensor information, and the control unit 150 is set to start the driving to run the intake air fans 16 of the intake air ports 101i, 103i and the purification unit 15 in a case where any one of the various types of sensor information exceeds a threshold set in advance. Here, in the present embodiment, a case of including three types of sensors, which are the light sensor 91, the human detection sensor 93, and the sound sensor 95, is described as an example; however, it is not limited thereto, and it is needless to say that it may be applicable to a case of including any one or two types.

As the light sensor 91, for example, a photoelectric sensor that detects a brightness in accordance with ON and OFF of a lighting in the dialysis facility R is employed and disposed, and assuming that the dialysis treatment is started once receiving luminosity information exceeding a threshold detecting that the lighting is ON, the control unit 150 starts the driving of the intake air fans 16 of the intake air ports 101i, 103i and the purification unit 15.

As the human detection sensor 93, for example, an infrared sensor that perceives the patient P using the bed B, a doctor who performs the input operation of the operation display unit 110, and the like is employed and disposed, and assuming that the dialysis treatment is started once receiving human detection information that determines it is the perception, the control unit 150 starts the driving of the intake air fans 16 of the intake air ports 101i, 103i and the purification unit 15.

As the sound sensor 95, a microphone, an amplifier, or the like that detects a conversation of the patient P using the bed B, the doctor who performs the input operation of the operation display unit 110, and the like and activity sounds such as installation sound of equipment is disposed, and assuming that the dialysis treatment is started once receiving the sound information, the control unit 150 starts the driving of the intake air fans 16 of the intake air ports 101i, 103i and the purification unit 15.

Particularly, the control unit 150 can also make adjustment such as increasing the processing capability by increasing an air amount sucked by the intake air fans 16 of the intake air ports 101i, 103i in accordance with the magnitude of the sound information of the sound sensor 95, and for example, it is possible to perform setting to forcibly perform suction processing of an airborne droplet spread during a conversation and the like depending on whether the volume of the sound information exceeds 60 dB based on a noise level of 45 dB in a normal hospital facility (Healthcare Engineering Association of Japan: Hospital Air Conditioning Design Guideline) and around a noise level of 60 dB in a normal conversation. Note that, it is possible to perform setting not to start the driving in a case of exceeding a noise level equal to or greater than a predetermined level to prevent a reaction to warning sound and notification sound during the treatment.

Additionally, this sound sensor 95 may be set in a direction to the patient P as a directional microphone so as to purify the treatment environment into clean air in conjunction with the conversation and the like effectively.

Thus, the blood purification apparatus 100 can automatically intensify the purification capability in a case where there is a high possibility that the spread of viruses and the like occurs more often like a case of a cough, a sneeze, and a loud voice of a person in the room including the patient P.

### <Effect of Ninth Embodiment>

Thus, in the blood purification apparatus 100 of the present embodiment, in addition to the operation and effect of the above-described embodiment, it is possible to automatically start the driving of the intake air fans 16 of the intake air ports 101i, 103i and the like and run the purification processing in accordance with the treatment environment, and it is possible to execute the purification processing of the treatment environment while suppressing occurrence of the noise and the electric power consumption along with the driving.

### <Tenth Embodiment>

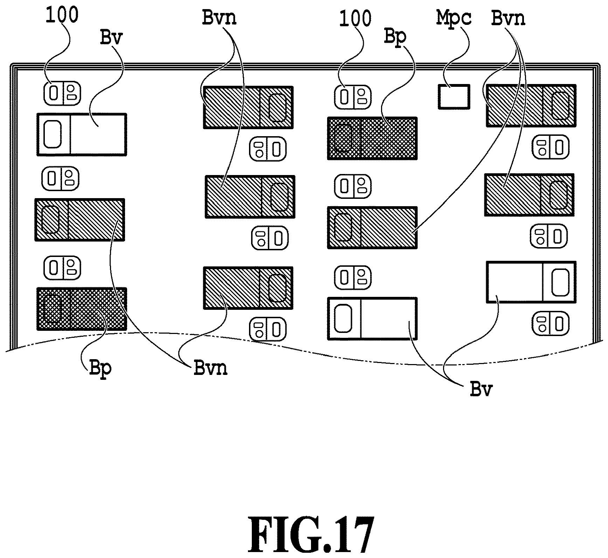

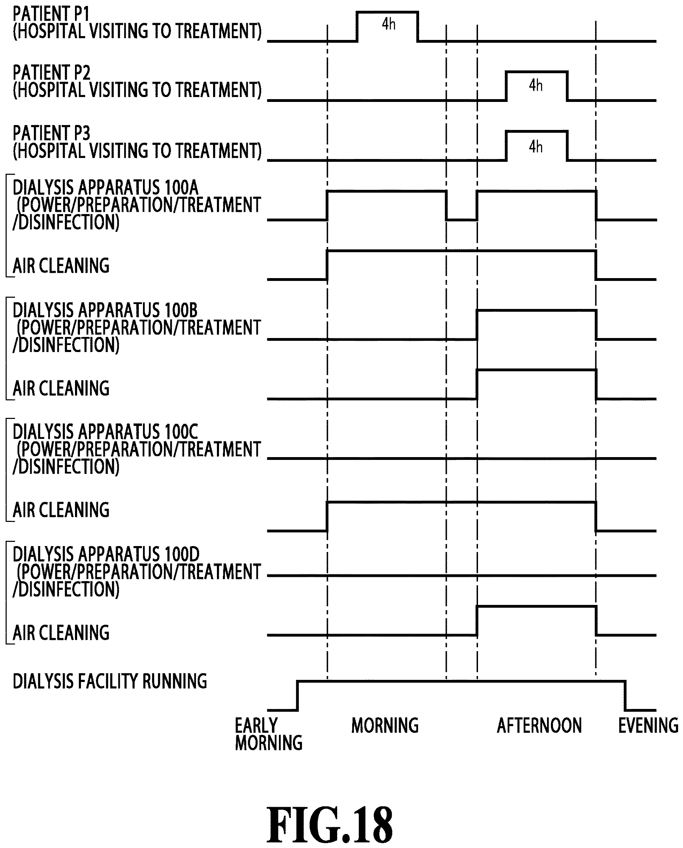

Figs. 17 and 18 are diagrams illustrating an embodiment of a blood purification management system according to the present invention.

In Fig. 17, with other blood purification apparatuses 100, the blood purification apparatus 100 is connected with wire (wireless connection may be applicable) to a management device Mpc disposed in the dialysis facility (treatment room) R in which the multiple beds B of the patients P are arranged. For example, the management device Mpc functions as a management system that manages all the running schedules of the individual blood purification apparatuses 100 including a treatment history and a treatment schedule of each patient P (bed B) by transmitting and receiving a control signal to and from the blood purification apparatuses 100 disposed in the dialysis facility R. This management device Mpc is, for example, constructed by a workstation in which a CPU executes control processing in accordance with various types of setting information based on a control program stored in a memory and makes an output on a monitor, and is constructed to a system that allows the blood purification apparatuses 100 of the corresponding beds B (patients P) connected through a local network can efficiently perform the dialysis treatment processing in the entire dialysis facility R. That is, the management device Mpc forms the blood purification management system.

The management device Mpc is configured to transmit and receive the control signal mutually with each control unit 150 of the blood purification apparatus 100 to cooperate therewith, and is also configured to be able to make not only an operation input from the management device Mpc but also to make an operation input from the operation display unit 110 of the individual blood purification apparatus 100.

Specifically, the management device Mpc is configured to transmit a dialysis control signal to the blood purification apparatus 100 beside the bed B of the patient P undergoing the dialysis treatment in the dialysis facility R and start the dialysis treatment processing and the air purification processing, and additionally to start the purification processing of the ambient air by transmitting a purification control signal to the control unit 150 of the blood purification apparatus 100 beside an adjacent vacant bed B around the bed B of the patient P.

For example, as illustrated in Fig. 17, the management device Mpc is configured to start the dialysis treatment processing and the air purification processing by transmitting a dialysis execution command to the control unit 150 of the blood purification apparatus 100 beside a bed Bp of the patient P in the dialysis facility R, and in parallel with this, not only for the patient bed Bp, configured to start the purification processing of the ambient air by transmitting the purification control signal also to the control unit 150 of the blood purification apparatus 100 beside a vacant bed Bvn adjacent to the patient bed Bp.

In this case, as illustrated in Fig. 18, for example, in a case where four hours of the dialysis treatment is each provided to one patient P1 in the morning and to two patients P2 and P3 in the afternoon, the management device Mpc properly executes the driving control of the blood purification apparatus 100 according to a schedule of the dialysis treatment within a period of time of the running from opening to closing of the dialysis facility R.

To be specific, blood purification apparatuses 100A and 100C of the bed Bp for the patients P1 and P2 and the adjacent vacant bed Bvn run from power-on (ON) to shutoff (OFF), while the blood purification apparatus 100Abeside the bed Bp used by the patients P1 and P2 receives the dialysis control signal at the time according to the schedules of the dialysis treatment of the two patients in the morning and the afternoon and executes the various types of required processing, and on the other hand, the blood purification apparatus 100C beside the adjacent vacant bed Bvn receives the purification control signal in the start of the dialysis treatment and drives the purification unit 15 with the intake air fans 16 of the intake air ports 101i, 103i to start the purification processing of the ambient air in a wide range including the surrounding bed Bvn around the bed Bp in use and continue until the dialysis treatment ends and the power is shut off.

Additionally, in a case where four hours of the dialysis treatment is provided to the one patient P3 in the afternoon, the driving control of the blood purification apparatus 100 is properly executed according to the schedule of the dialysis treatment.

To be specific, blood purification apparatuses 100B and 100D of the bed Bp for the patient P3 and the adjacent vacant bed Bvn run from power-on (ON) to shutoff (OFF), while the blood purification apparatus 100B beside the bed Bp used by the patient P3 receives the dialysis control signal at the time according to the schedule of the dialysis treatment of the one patient in the afternoon and executes the various types of required processing, and on the other hand, the blood purification apparatus 100D beside the adjacent vacant bed Bvn receives the purification control signal in the start of the dialysis treatment and drives the purification unit 15 with the intake air fans 16 of the intake air ports 101i, 103i to start the purification processing of the ambient air in a wide range including the surrounding bed Bvn around the bed Bp in use and continue until the dialysis treatment ends and the power is shut off.

### <Effect of Tenth Embodiment>

Thus, in the dialysis facility R of the present embodiment, it is possible to establish cooperation only by setting the running of the blood purification apparatus 100 used by the patient P with no time and effort of individually setting various driving conditions and the like, and it is possible to provide the dialysis treatment in an ambient environment of the already purified air subjected to the sterilization and inactivation processing of viruses and bacteria in a wide range including the ambient air adjacent to the vicinity of the bed B.

### <Summary of Embodiments>

Next, matters specifying the invention forming the present invention are described continuously by incorporating the reference signs and the like in the above-described embodiments. Note that, each reference sign and the like described below are merely for describing a constituent within the scope of a claim by incorporating the descriptions of the embodiments, and it is needless to say that it is not intended to limit the constituent to a specific member and the like.

"1" A blood purification apparatus including a blood purification unit (a dialyzer, a blood pump 123, and the like) that executes blood purification treatment, comprising:
   an air purification unit (an intake air fan 16 and a purification unit 15) that discharges sucked external air after purification; and
   a control unit (150) that controls the blood purification unit and the air purification unit, wherein
   the control unit drives the air purification unit in conjunction with running of the blood purification unit and allows the air purification unit to purify air around a patient subjected to the purification of blood by the blood purification unit.
"2" The blood purification apparatus according to "1", wherein
   the air purification unit is arranged in the middle of a ventilation route (an upper space 101 and a lower space 103) that sucks and discharges external air so as to pass through a heating portion (printed substrates 153 and 155) in an apparatus main body including the blood purification unit.
"3" The blood purification apparatus according to "1", wherein
   the air purification unit is formed as a separate apparatus (an air purification apparatus 20 or 30) from an apparatus main body including the blood purification unit and is connected to the control unit with wire or wireless so as to be able to cooperate.
"4" The blood purification apparatus according to any one of "1" to "3", wherein
   out of an intake air port (101i, 103i) and an exhaust air port (101o, 103o) of the external air in the blood purification unit, at least the intake air port is disposed near a head of the patient.
"5" The blood purification apparatus according to "4", wherein
   a member (a wind direction plate 71 or a bellows 81) that changes an air flowing direction is disposed in one of or both the intake air port and the exhaust air port.
"6" The blood purification apparatus according to "4" or "5", wherein
   the intake air port and the exhaust air port are arranged in a front surface (100f) and a back surface (100b) of an apparatus main body including the blood purification unit or in two side surfaces (100s) of the apparatus main body.
"7" The blood purification apparatus according to any one of "4" to "6", wherein
   an extending ventilation route (a flow route unit 40) extending the ventilation route to outside is disposed in one of or both the intake air port and the exhaust air port.
"8" The blood purification apparatus according to "7", wherein
   a pathway member (a flow route unit 50) forming the extending ventilation route is created from a deformable material to be able to arbitrarily adjust an extending position of one of or both the intake air port and the exhaust air port.
"9" The blood purification apparatus according to any one of "1" to "8", wherein
   the control unit is provided with a function to set a running schedule of the blood purification unit and drives the air purification unit in conjunction with the running schedule.
"10" The blood purification apparatus according to any one of "1" to "9", further comprising:
   at least one or more of a light sensor that detects light, a human detection sensor that perceives a human, and a sound sensor that detects sound, wherein
   driving of the air purification unit is controlled based on a signal of the sensor.
"11" The blood purification apparatus according to "10", wherein
   suction capability of the air purification unit is controlled based on a magnitude of a sound signal detected by the sound sensor.
"12" A blood purification management system (a management device Mpc) to which a plurality of the blood purification apparatuses according to any one of" 1" to "11" described above are connected and that manages a running schedule, wherein
   a control signal to drive the air purification function is transmitted to one or more separate blood purification apparatuses disposed around the blood purification apparatus in which the blood purification unit runs.

In a case of the above-described "1" to "12", it is possible to clean the air around the patient with no time and effort in a simple and easy manner by performing not only the blood purification but also the air purification in the treatment environment.

In a case of the above-described "7" and "8", it is possible to set intake and exhaust air ports to arbitrary positions and to achieve the purification of the ambient air in the treatment environment effectively.

In a case of the above-described "9", it is possible to automatically perform the air purification in accordance with the running schedule of the blood purification apparatus and to purify the ambient air in the treatment environment unconsciously.

In a case of the above-described "10" and "11", it is possible to achieve the purification of the ambient air in the treatment environment effectively in conjunction with a person related to the treatment and additionally in conjunction with a conversation and the like of the person.

In a case of the above-described "12", it is possible to automatically perform the air purification in cooperation with the surrounding disposed blood purification apparatus in accordance with the running schedule of the blood purification apparatus and to purify the ambient air in a wider range in the treatment environment unconsciously.

The scope of the present invention is not limited to the exemplified embodiment illustrated and described and includes also all the embodiments that provide an effect equivalent to an object of the present invention. Additionally, the scope of the present invention is not limited to a combination of the characteristics of the invention defined by each claim and can be defined by any desired combination of specific characteristics out of all the respective characteristics disclosed.

### Reference Signs List

15...... purification unit
16......intake air fan
17......ultraviolet rays light emitter
18......photocatalyst-supported filter
20, 30...... air purification apparatus
21, 41, 101i, 103i......intake air port
23, 33,43, 53, 101o, 103o......exhaust air port
40, 50......flow route unit
52......exhaust air duct
71......wind direction plate
81......bellows
91......light sensor
93......human detection sensor
95......sound sensor
100, 100A to 100D......blood purification apparatus
100b......apparatus back surface
100f......apparatus front surface
100s ...... apparatus side surface
101......upper space
103......lower space
150......control unit
151, 153, 155......printed substrate
160......support pole
B, Bp, Bv, Bvn......bed
C......shielding curtain
Mpc......management device
P, P1 to P3......patient
R......dialysis facility

## Claims

1. A blood purification apparatus including a blood purification unit that executes blood purification treatment, comprising:
an air purification unit that sucks external air and discharges the sucked external air after purification; and
a control unit that controls the blood purification unit and the air purification unit, wherein
the control unit drives the air purification unit in conjunction with running of the blood purification unit and allows the air purification unit to purify air around a patient subjected to the purification of blood by the blood purification unit.

2. The blood purification apparatus according to claim 1, wherein
the air purification unit is arranged in the middle of a ventilation route that sucks and discharges external air so as to pass through a heating portion in an apparatus main body including the blood purification unit.

3. The blood purification apparatus according to claim 1, wherein
the air purification unit is formed as a separate apparatus from an apparatus main body including the blood purification unit and is connected to the control unit with wire or wireless so as to be able to cooperate.

4. The blood purification apparatus according to any one of claims 1 to 3, wherein
out of an intake air port and an exhaust air port of the external air in the blood purification unit, at least the intake air port is disposed near a head of the patient.

5. The blood purification apparatus according to claim 4, wherein
a member that changes an air flowing direction is disposed in one of or both the intake air port and the exhaust air port.

6. The blood purification apparatus according to any one of claims 1 to 5, wherein
an intake air port and an exhaust air port are arranged in a front surface and a back surface of an apparatus main body including the blood purification unit or in two side surfaces of the apparatus main body.

7. The blood purification apparatus according to any one of claims 4 to 6, wherein
an extending ventilation route extending a ventilation route, which is between the intake air port and the exhaust air port in an apparatus main body including the blood purification unit, to outside is disposed in one of or both the intake air port and the exhaust air port.

8. The blood purification apparatus according to claim 7, wherein
a pathway member forming the extending ventilation route is created from a deformable material to be able to arbitrarily adjust an extending position of one of or both the intake air port and the exhaust air port.

9. The blood purification apparatus according to any one of claims 1 to 8, wherein
the control unit is provided with a function to set a running schedule of the blood purification unit and drives the air purification unit in conjunction with the running schedule.

10. The blood purification apparatus according to any one of claims 1 to 9, further comprising:
at least one or more of a light sensor that detects light, a human detection sensor that perceives a human, and a sound sensor that detects sound, wherein
driving of the air purification unit is controlled based on a signal of the sensor.

11. The blood purification apparatus according to claim 10, wherein
suction capability of the air purification unit is controlled based on a magnitude of a sound signal detected by the sound sensor.

12. A blood purification management system to which a plurality of the blood purification apparatuses according to any one of claims 1 to 11 described above are connected and that manages a running schedule, wherein
a control signal to drive the air purification unit is transmitted to one or more separate blood purification apparatuses disposed around the blood purification apparatus in which the blood purification unit runs.
